# EUROPEAN PATENT SPECIFICATION

(11) **EP 0 773 781 B1**
(45) Date of publication and mention of the grant of the patent: **22.10.2003**
(21) Application number: 95927856.5
(22) Date of filing: 04.08.1995
(51) Int. Cl.: A61K 9/16, A61K 9/22

(54) **SOLID DELIVERY SYSTEMS FOR CONTROLLED RELEASE OF MOLECULES INCORPORATED THEREIN AND METHODS OF MAKING SAME**
FESTE VERABREICHUNGSSYSTEME ZUR GESTEUERTEN FREISETZUNG VON DARIN EINGEBAUTEN MOLEKÜLEN SOWIE VERFAHREN ZU DEREN HERSTELLUNG
SYSTEMES D'ADMINISTRATION DE SUBSTANCES SOLIDES, POUR LA LIBERATION CONTROLEE DE MOLECULES INCORPOREES DANS CES SUBSTANCES ET PROCEDES DE FABRICATION DE CES SYSTEMES

(30) Priority: 04.08.1994 GB 9415810; 02.12.1994 US 349029
(43) Date of publication of application: 21.05.1997
(60) Divisional application: 01116637.8
(73) Proprietor: Elan Drug Delivery Limited, Nottingham, NG11 6JS (GB)
(72) Inventor: ROSER, Bruce, Joseph, Cambridge CB3 9LW (GB); COLACO, Camilo, Cambridge CB2 2JN (GB); JERROW, Mohamed, Abdel, Zahra, Aberdeen AB1 24H (GB); BLAIR, Julian, Alexander 27 Landcliffe Road, Cambridgeshire PE17 6JF (GB); KAMPINGA, Jaap, NL-9731 MJ Groningen (NL); WARDELL, James, Lewis, Aberdeen AB2 4NY (GB); DUFFY, John, Alistair, Aberdeen AB2 4DS (GB)
(74) Representative: Perry, Robert Edward
(86) International application number: GB9501861
(87) International publication number: WO96003978

(56) References cited:
- WO-A-88/08298
- WO-A-90/11756
- WO-A-91/18091
- WO-A-93/10758
- WO-A-94/22423
- DE-B- 1 080 265
- PHARMACEUTICAL RESEARCH, vol. 6, no. 11, November 1989 NEW YORK (US), pages 958-960, XP 000121530 L. HAHN ET AL. 'SOLID SURFACTANT SOLUTIONS OF ACTIVE INGREDIENTS IN SUGAR ESTERS'

## Description

### Field of the Invention

The present invention relates generally to solid delivery systems for storage, distribution and controlled delivery of molecules and, more specifically, to solid dose delivery systems comprising a vitreous vehicle and guest substances. Methods of making the delivery systems and methods of use thereof are also provided.

### Background of the Invention

Solid delivery systems are useful in a wide, variety of applications such as controlled release of labile molecules, particularly bioactive materials such as pharmaceutical agents, enzymes, vaccines and biological control agents such as fertilisers, pesticides and pheromones.

Solid dose delivery of bioactive materials to biological tissues such as mucosal, dermal, ocular, subcutaneous, intradermal and pulmonary offers several advantages over previous methods such as topical applications of liquids, transdermal administration via so-called "patches" and hypodermic injection. Solid dose delivery can be by direct transdermal delivery of the solid dose which reduces the risk of infection by eliminating the use of conventional needles and syringes and provides for more accurate dosing than multidose vials, and minimizes or eliminates the discomfort which often attends hypodermic injection. Several solid dose delivery systems have been developed including those utilizing transdermal and ballistic delivery devices.

A variety of formulations have been provided for administration in aerosolized form to mucosal surfaces, particularly "by-inhalation" (naso-pharyngeal and pulmonary). Compositions for by-inhalation pharmaceutical administration generally comprise a liquid formulation of the pharmaceutical agent and a device for delivering the liquid in aerosolized form. United States Patent No. 5,011,678 describes suitable compositions containing a pharmaceutically active substance, a biocompatible amphiphilic steroid and a biocompatible (hydro/fluoro) carbon propellant. United States Patent No. 5,006,343 describes suitable compositions containing liposomes, pharmaceutically active substances and an amount of alveolar surfactant protein effective to enhance transport of the liposomes across a pulmonary surface.

One drawback to the use of aerosolized formulations is that maintenance of pharmaceutical agents in aqueous suspensions or solutions can lead to aggregation and loss of activity and bioavailability. The loss of activity can be partially prevented by refrigeration; however, this limits the utility of these formulations. This is particularly true in the case of peptides and hormones. For instance, synthetic gonadotropin releasing hormone (GnRH) analogs, such as the agonist nafarelin or the antagonist ganirelex, are designed for high potency, increased hydrophobicity and membrane binding. The compounds have sufficient hydrophobic character to aggregate in aqueous solution and to form an ordered structure that increases in viscosity with time. Thus bioavailability in nasal or pulmonary formulations may be prohibitively low. The use of powdered formulations overcomes many of these drawbacks. The requisite particle size of such powders is 0.5-5 microns in order to attain deep alveolar deposition in pulmonary delivery. Unfortunately, powders of such particle size tend to absorb water and clump, thus diminishing deposition of the powder in the deep alveolar spaces. Although powders with larger particle size are suitable for delivery to the naso-pharynx region, the tendency of powders to clump decreases the available particle surface area for contact with, and absorption through, these membranes. Devices which disaggregate clumps formed by electrostatic interactions are currently in use (e.g., the Turbohaler™); however, these do not disaggregate moisture-induced clumps. It would be advantageous to have powders which do not absorb moisture and clump, thus increasing the effective pulmonary concentration of the drug.

WO 95/24183 is in the state of the art according to Article 54(3) EPC. It discloses methods and compositions for the pulmonary delivery of insulin. If the insulin composition contains a polyol, citrate is also present.

### Summary of the Invention

According to the present invention, in a particulate composition, for therapeutic use, suitable for administration by inhalation, the particles consist of a solid solution comprising a therapeutic agent and a glass-forming carbohydrate, higher than a monosaccharide, and that is capable of stabilising the agent during spray-drying and storage, with the proviso that when the therapeutic agent is insulin, the solution does not comprise citrate.

### Brief Description of the Drawings

Figure 1 is a graph depicting typical particle size distribution of micronized trehalose glass powder suitable for administration by inhalation. Figure 1 is described in Example 2.
Figure 2A is a graph depicting the narrow particle size distribution for trehalose/molecular water pump buffer salt (MWPB) glass powder. Figure 2B is a graph depicting the water absorption of various trehalose/MWPB and trehalose/chloride glass powders after storage at ambient temperature and different relative humidities. Figure 2B depicts 51% relative humidity and MWPB (■), 80% relative humidity and MWPB (|), 51% relative humidity and chloride (□) and 80% relative humidity and chloride (X). Figure 2 is described in Example 2.
Figure 3 is a graph depicting the narrow particle size distribution for trehalose glass powder obtained by spray-drying in a Lab-plant spray dryer. Figure 3 is described in Example 2.
Figure 4 is a graph depicting a comparison of the sharp particle size distribution for trehalose glass powders (0.5M trehalose/0.5M calcium lactate) prepared with two different spray-dryers (Lab-Plant (□) and Buchi ( 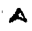 ), as indicated). Figure 4 is described in Example 2.

### Detailed Description of the Invention

The present invention comprises solid dose delivery systems comprising solid dose delivery vehicles and guest substances. The delivery systems are formulated to provide precise delivery rates of the guest substances incorporated therein. The delivery systems are particularly suitable for delivery of bioactive molecules to animals including humans.

Also encompassed by the invention are methods of delivery of therapeutic agents including, but not limited to, mucosal, oral, topical, subcutaneous and intradermal, intramuscular, intravenous and by-inhalation administration.

The invention also encompasses methods of making the delivery systems.

"Solid dose" as used herein, means that a guest substance incorporated in the vehicle is in solid rather than liquid form and the solid form is the form used for delivery. Guest substances are those molecules, macromolecules and macromolecular assemblies, synthetic and natural, and cellular fractions, live and dead cells, bacteria and viruses and other actives incorporated into the vehicle; a wide variety of guest substances are suitable for use herein and are described below. By "effective amount" of guest substance, is meant an amount to achieve the affect desired. For instance, with a bioactive material, an effective amount is one which effects the desired physiological reaction. The vehicle is in solid form and is amorphous or glassy in nature. Other additives, buffers, dyes etc. may be incorporated into the delivery systems. As used herein, the term "vehicle" includes all the glass-forming substances embodied in the claimed invention. The term "delivery system(s)" includes the solid dose forms comprising the vehicles and guest substances. Delivery systems formed from specific vehicles are given distinct names as indicated, unless otherwise indicated, the term delivery system encompasses each of these.

In one embodiment, the invention relates to solid dose systems with rapid release rates of the guest substances. In this embodiment, the vehicle is a SP. It has now been found that SPs can be processed to obtain powders with homogeneous distribution of particle sizes in the form of either microspheres or needles. The SPs can also be processed to form macroscopic delivery forms suitable for formulation of implantable devices. A wide variety of dose forms and methods of making the dose forms are described herein. These SPs have been found to be particularly useful where otherwise denaturing conditions would render impossible the formulation of solid dosage forms of bioactive materials. In particular, such conditions include elevated temperatures (those above which the bioactive material is otherwise denatured) and the presence of organic solvents.

In another embodiment, the invention relates to solid dose systems with novel defined and controllable release rates of the guest substances. In this embodiment, the vehicle is an organic carboxylate glass. It has now been found that organic carboxylates form stable amorphous vehicles by solvent evaporation. These organic glasses release incorporated guest substances at precisely defined rates depending on the composite carboxylate anion and metal cation used. Like the vehicles comprising SPs, these glasses can be processed, either singly or in mixtures with other organic carboxylates and/or SPs and/or HDCs, to obtain powders with homogeneous particle size distribution, in the form of microspheres, needles and/or implantable devices to form a wide variety of macroscopic delivery forms.

The present invention encompasses compositions and methods of making the compositions. Although singular forms may be used, more than one vehicle, more than one guest substance and more than one additive may be present. Determination of the effective amounts of these compounds is within the skill of one in the art.

### Stabilizing Polyol Delivery Systems

The invention encompasses solid dose delivery systems in which the delivery vehicle comprises a stabilizing polyol. These are termed "SP delivery systems". It has now been found that the SP delivery systems can be processed to a wide variety of solid dose forms particularly suited to therapeutic administration of guest substances.

SPs include, but are not limited to, carbohydrates. As used herein, the term "carbohydrates" includes, but is not limited to, monosaccharides, disaccharides, trisaccharides, oligosaccharides and their corresponding sugar alcohols, polysaccharides and chemically modified carbohydrates such as hydroxyethyl starch and sugar copolymers (Ficoll). Both natural and synthetic carbohydrates are suitable for use herein. Synthetic carbohydrates include, but are not limited to, those which have the glycosidic bond replaced by a thiol or carbon bond. Both D and L forms of the carbohydrates may be used. The carbohydrate may be non-reducing or reducing. Suitable vehicles are those in which a guest substance can be dried and stored without losses in significant activity by denaturation, aggregation or other mechanisms. Prevention of losses of activity can be enhanced by the addition of various additives such as inhibitors of the Maillard reaction as described below. Addition of such inhibitors is particularly preferred in conjunction with reducing carbohydrates.

Reducing carbohydrates suitable for use in the present invention are those known in the art and include, but are not limited to, glucose, maltose, lactose, fructose, galactose, mannose, maltulose, iso-maltulose and lactulose.

Non-reducing carbohydrates include, but are not limited to, trehalose, raffinose, stachyose, sucrose and dextran. Other useful carbohydrates include non-reducing glycosides of polyhydroxy compounds selected from sugar alcohols and other straight chain polyalcohols. The sugar alcohol glycosides are preferably monoglycosides, in particular the compounds obtained by reduction of disaccharides such as lactose, maltose, lactulose and maltulose. The glycosidic group is preferably a glucoside or a galactoside and the sugar alcohol is preferably sorbitol (glucitol). Particularly preferred carbohydrates are maltitol (4-O-β-D-glucopyranosyl-D-glucitol), lactitol (4-O-β-D-galactopyranosyl-D-glucitol), palatinit (a mixture of GPS, α-D-glucopyranosyl-1→6-sorbitol and GPM, α-D-glucopyranosyl-1→6-mannitol), and its individual sugar alcohols, components GPS and GPM.

Preferably, the SP is a carbohydrate that exists as a hydrate, including trehalose, lactitol and palatinit. Most preferably, the SP is trehalose. It has now been found that, surprisingly, solid dose delivery systems containing certain sugar hydrates like trehalose lack the "stickiness" or "tackiness" of solid dose forms containing other carbohydrates. Thus, for manufacture, packaging and administration, trehalose is the preferred SP.

Trehalose, (α-D-glucopyranosyl-α-D-glucopyranoside), is a naturally occurring, non-reducing disaccharide which was initially found to be associated with the prevention of desiccation damage in certain plants and animals which can dry out without damage and can revive when rehydrated. Trehalose has been shown to be useful in preventing denaturation of proteins, viruses and foodstuffs during desiccation. See U.S. Patent Nos. 4,891,319; 5,149,653; 5,026,566; Blakeley et al. (1990) Lancet 336:854-855; Roser (July 1991) Trends in Food Sci. and Tech. 166-169; Colaco et al. (1992) Biotechnol, Internat., 345-350; Roser (1991) BioPharm. 4:47-53; Colaco et al. (1992) Bio/Tech. 10:1007-1011; and Roser et al. (May 1993) New. Scientist, pp. 25-28.

Other SPs suitable for use herein are described for instance in, WO 91/18091, 87/00196 and U.S. Patent nos. 4,891,319 and 5,098,893 which describe the use of polyols as glasses for stabilizing molecules during drying and storage for reconstitution before use. The solid dosage forms encompassed by the present invention have now been found to be suitable for use directly, as delivery systems for controlled release of incorporated guest substances. Additionally, these polyols can be used in combination with other amorphous matrices to yield delivery systems which have now been found to have a wide range or release rates and characteristics which are readily and accurately controllable to produce unique solid dose systems.

It has also now been found that guest substances preferentially soluble in organic solvents can be dried in trehalose from an organic/aqueous solvent mixture to give a conformulation that is now readily reconstituted in aqueous solvents. The present invention encompasses solid dose systems obtained in this manner. Methods of making the dried material and compositions obtained thereby are provided by the invention. The guest substance is dissolved in an organic/aqueous solvent in combination with an effective amount of trehalose and then dried. This gives a solid solution, emulsion, suspension or coacervate of the guest substance in a trehalose glass which then readily dissolves in an aqueous solution to give a finely dispersed suspension of the insoluble guest substance. It has now been shown that the immunosuppressant CSA (which is poorly soluble in water and normally administered as an oil emulsion) in a solution of trehalose in a 1:1 ethanol:water mixture can be dried to give a clear glass of trehalose containing CSA. This glass can be milled to give a free flowing powder, which can also be tabletted, which when added to water dissolves instantaneously to give a finely dispersed suspension of CSA in water.

### Other Components in the Delivery Systems

### Other glasses

As discussed below, the delivery systems may further contain at least one physiologically acceptable glass. Suitable glasses include, but are not limited to, carboxylate, phosphate, nitrate, sulfate, bisulfate, HDCs and combinations thereof. Carboxylates have previously been used where slowly water soluble glasses are required as many of these are only poorly soluble in water. Suitable such glasses include, but are not limited to, those described in PCT/GB 90/00497. However, the formation of these carboxylate glasses has previously only been done by quenching of the melt. The elevated temperature necessary to melt the carboxylates severely limits the carboxylates that can be used to form vitreous delivery vehicles, particularly in the case of bioactive materials which tend to be heat labile. We have now found, surprisingly, that carboxylate glasses can be easily formed by evaporation of a solvent containing the glass-forming metal carboxylate and guest substance to be incorporated. The invention thus encompasses methods of making solid dose vehicles and systems comprising dissolving a carboxylate component in a suitable solvent therefor and evaporating the solvent to yield a vitreous glass. Mixtures of carboxylates can be used as can mixtures of other glass-forming components to produce novel delivery systems which are encompassed by the present invention.

The delivery systems may also be coated with one or more layers of a physiologically acceptable glass having a predetermined solution rate. This is especially effective for pulsatile release of guest substances. The composition may further contain other water soluble and biodegradable glass formers. Suitable glass formers include, but are not limited to, lactide and lactide/glycolide copolymers, glucuronide polymers and other polyesters, polyorthoesters, and polyanhydrides.

### Guest substances

Examples of types of guest substances that may be used in the vehicle and methods of the invention include industrial chemicals such as dyes and perfumes and medicinal or agricultural bioactive materials suitable for use in vivo and in vitro. Suitable bioactive materials include, but are not limited to, pharmaceutical agents, therapeutic and prophylactic agents and agrochemicals such as pesticides and pheromones.

Suitable pharmaceutical agents, include, but are not limited to, antiinflammatory drugs, analgesics, antiarthritic drugs, antispasmodics, antidepressants, antipsychotics, tranquilizers, antianxiety drugs, narcotic antagonists, antiparkinsonism agents, choiinergic agonists, chemotherapeutic drugs, immunosuppressive agents, antiviral agents, antibiotic agents, appetite suppressants, antiemetics, anticholinergics, antihistaminics, antimigraine agents, coronary, cerebral or peripheral vasodilators, hormonal agents, contraceptives, antithrombotic agents, diuretics, antihypertensive agents, cardiovascular drugs, opioids, and the like.

Suitable therapeutic and prophylactic agents include, but are not limited to, any therapeutically effective biological modifier. Such modifiers include, but are not limited to, subcellular compositions, cells, bacteria, viruses and molecules including, but not limited to, lipids, organics, proteins and peptides (synthetic and natural), peptide mimetics, hormones (peptide, steroid and corticosteroid), D and L amino acid polymers, oligosaccharides, polysaccharides, nucleotides, oligonucleotides and nucleic acids, including DMA and RNA, protein-nucleic acid hybrids, small molecules and physiologically active analogs thereof. Further, the modifiers may be derived from natural sources or made by recombinant or synthetic means and include analogs, agonists and homologs.

As used herein "protein" refers also to peptides and polypeptides. Such proteins include, but are not limited to, enzymes, biopharmaceuticals, growth hormones, growth factors, insulin, monoclonal antibodies, interferons, interleukins and cytokines.

Organics include, but are not limited to, pharmaceutically active chemicals. For instance, representative organics include, but are not limited to, vitamins, neurotransmitters, antimicrobials, antihistamines, analgesics and immunosuppressants.

Suitable steroid hormones include, but are not limited to, corticosteroids, estrogen, progesterone, testosterone and physiologically active analogs thereof. Numerous steroid hormone analogs are known in the art and include, but are not limited to, estradiol, SH-135 and tamoxifen. Many steroid hormones such as progesterone, testosterone and analogs thereof are particularly suitable for use in the present invention as they are not absorbed transdermally and, with the exception of a few analogs, are destroyed upon oral administration by the so-called hepatic first pass mechanism.

As used herein, "nucleic acids" includes any therapeutically effective nucleic acids known in the art including, but not limited to, DNA, RNA and physiologically active analogs thereof. The nucleotides may encode single genes or may be any vector known in the art of recombinant DNA including, but not limited to, plasmids, retroviruses and adeno-associated viruses. Preferably, the nucleotides are administered in the powder form of the solid dose system.

Compositions comprising solid dose delivery systems containing prophylactic bioactive materials and carriers therefore are further encompassed by the invention. Preferable compositions include immunogens such as for use in vaccines. Preferably, the compositions contain an immunogenic amount of the immunogen effective for either immunization or booster inoculation.

Suitable immunogens include, but are not limited to, live and attenuated viruses, nucleotide vectors encoding antigens, bacteria, antigens, antigens plus adjuvants, and haptens coupled to carriers. Particularly preferred are immunogens effective in causing an immune response against diphtheria, tetanus, pertussis, botulinum, cholera, Dengue, Hepatitis A, C and E, hemophilus influenza b, herpes virus, *Helicobacterium pylori,* influenza, Japanese encephalitis, meningococci A, B and C, measles, mumps, papilloma virus, pneumococci, polio, rubella, rotavirus, respiratory syncytial virus, Shigella, tuberculosis, yellow fever and combinations thereof.

Immunogens may also be produced by molecular biology techniques to produce recombinant peptides or fusion proteins containing one or more portions of a protein derived from a pathogen. For instance, fusion proteins containing the antigen of interest and the B subunit of cholera toxin have been shown to induce an immune response to the antigen of interest. Sanchez et al. (1989) Proc. Natl. Acad. Sci. USA 86:481-485.

Preferably, the immunogenic composition contains an amount of an adjuvant sufficient to enhance the immune response to the immunogen. Suitable adjuvants include, but are not limited to, aluminum salts, squalene mixtures (SAF-1), muramyl peptide, saponin derivatives, mycobacterium cell wall preparations, monophosphoryl lipid A, mycolic acid derivatives, nonionic block copolymer surfactants, Quil A, cholera toxin B subunit, polyphosphazene and derivatives, and immunostimulating complexes (ISCOMs) such as those described by Takahashi et al. (1990) Nature 344:873-875. For veterinary use and for production of antibodies in animals, mitogenic components of Freund's adjuvant can be used.

As with all immunogenic compositions, the immunologically effective amounts of the immunogens must be determined empirically. Factors to be considered include the immunogenicity, whether or not the immunogen will be complexed with or covalently attached to an adjuvant or carrier protein or other carrier, route of administration and the number of immunizing doses to be administered. Such factors are known in the vaccine art and it is well within the skill of immunologists to make such determinations without undue experimentation.

Preferably, if the guest substance and/or vehicle contain carboxyl and amino, imino or guanidino groups, the delivery systems further comprise at least one physiologically acceptable inhibitor of the Maillard reaction in an amount effective to substantially prevent condensation of amino groups and reactive carbonyl groups in the composition.

The inhibitor of the Maillard reaction can be any known in the art. The inhibitor is present in an amount sufficient to prevent, or substantially prevent, condensation of amino groups and reactive carbonyl groups. Typically, the amino groups are present on the bioactive material and the carbonyl groups are present on the carbohydrate, or the converse. However, the amino and carbonyl groups may be intramolecular, within either the biological substance or the carbohydrate. Various classes of compounds are known to exhibit an inhibiting effect on the Maillard reaction and hence to be of use in the compositions described herein. These compounds are generally either competitive or noncompetitive inhibitors. Competitive inhibitors include, but are not limited to, amino acid residues (both D and L), combinations of amino acid residues and peptides. Particularly preferred are lysine, arginine, histidine and tryptophan. Lysine and arginine are the most effective. There are many known noncompetitive inhibitors. These include, but are not limited to, aminoguanidine and derivatives, are 4-hydroxy-5,8-dioxoquinoline derivatives and suitable Maillard inhibitors such as those in EP-A-O 433 679.

### Dosage Forms

In addition to the dosage forms described above, a variety of other dosage forms suitable for different uses are provided herein.

The invention encompasses delivery systems that are sized and shaped for penetration of the epidermis and are suitable for ballistic delivery. Suitable vehicle size is thus on the order of microns, preferably in the range of 1-5 microns in diameter and 5-150 microns in length, which allows penetration and delivery through the epidermis to subcutaneous and intradermal, intramuscular, intravenous tissues. It will be appreciated that, at this size, the delivery system may macroscopically appear to be in powder form, regardless of its configuration at the microscopic level.

Preferred configurations of the ballistic delivery systems are microneedles and microfibers. The manufacture of microfibers is relatively simple and economical and results in stable delivery systems comprised of the vehicle in glassy form and the guest substance. Additional stabilizers, buffers, glasses and polymers may also be added during processing as described herein. Many of the most labile biomolecules can withstand high temperatures (e.g., 60-100°C) when stabilized by drying in trehalose, provided that the majority of their surface is in contact with the vehicle. Temperatures of 70°C can be tolerated for over a month (Colaco et al. (1992) Bio/Technology 10:1007-1011) and higher temperatures for shorter periods. The results presented herein show that the fluorescent protein phycoerythrin dried in trehalose can be stored at 100°C for at least one month with no detectable loss of functional activity. Other vehicles give protection at lower temperatures than trehalose. The maximum temperature of protection must be determined empirically and is within the skill of one in the art without undue experimentation.

The microfibers prepared in accord with the principles of the present invention have a relatively high aspect ratio, i.e., length compared to diameter, preferably in the range of 1-5 microns in diameter and 5-150 microns in length. This high aspect ratio provides for enhanced "end on" penetration upon ballistic delivery, by the tendency of the microfibers to line up parallel to the barrel of the ballistic microinjector, as described in more detail below. Longer macrofibers may be injected using conventional impact ballistic devices or by trocar. Alternatively, macroscopic glass needles of sufficient intrinsic strength may be directly driven in through the skin for subcutaneous, intradermal or intramuscular administration of the guest substance.

Alternative preferred embodiments of the delivery systems include uniform microspheres, preferably with a narrow size distribution. This configuration is particularly useful when increased control of the depth of penetration of the delivery system is desirable. Such control would be useful, for example, for intradermal, intramuscular, intravenous delivery of vaccines to the basal layer of the epidermis, to bring antigen into proximity to the Langerhans cells of the skin to induce optimal immune responses.

The invention also encompasses hollow fibers for delivery of guest substances. By drawing down a hollow billet through a zone furnace which produces local softening of the vitreous vehicle, fine hollow needles can be formed. These needles can be filled with a finely powdered stabilized compound by introduction of the fine powder during the melting and drawing down process. The hollow fiber can also be made of thermoplastic, organic polymer and/or carbohydrate and/or HDC which may itself be slowly or rapidly water soluble and/or biodegradable.

An alternative embodiment of the delivery vehicle in the invention comprises a hollow vehicle comprised of poorly water soluble glass or plastic which is filled and optionally coated the delivery systems described herein.

In another embodiment of the invention, coformulations of vehicles and other poorly water soluble materials are included. For example, coformulations of vehicles with water-soluble glasses such as phosphate, nitrate or carboxylate glasses or biodegradable plastics such as lactide or lactide/glycolide copolymers will yield a more slowly eroding vehicle for delayed release of the bioactive material.

### Methods of Making the Delivery Systems

The invention further encompasses methods of making the solid dose systems. Providing the exposure time is limited, guest substances admixed in dry vehicles can be heated to fluidize the glass which can then be drawn or spun as a fiber without damage to the product. Fibers can either be drawn from a billet, cooled to solidify them and then wound onto a drum or they can be spun through fine holes in a rapidly rotating cylinder that is heated above the melting point of the vehicle. Being inherently brittle, these fibers can be readily cut, broken, crushed or chopped into short lengths to form long cylindrical rods or needles. By varying the diameter of the fibers produced, needles can be formed which vary from micro to macro needles, i.e., from thicknesses of a few microns to fractions of a millimeter. It has been found that cotton candy machines are suitable for use in preparing the finer diameter microfibers. Although the optimal conditions must be determined empirically for each vehicle, such determinations are well within the skill of one in the art.

To prepare microspheres of the present invention, several methods can be employed depending upon the desired application of the delivery vehicles. Suitable methods include, but are not limited to, spray drying, freeze drying, air drying, vacuum drying, fluidized-bed drying, milling, co-precipitation and super-critical fluid evaporation. In the case of spray drying, freeze drying, air drying, vacuum drying, fluidized-bed drying and super-critical fluid evaporation, the components (SP and/or HDC, and/or other glass former, guest substances, buffers etc.) are first dissolved or suspended in suitable solvents. In the case of milling, glasses formed from the components, either by solvent evaporation or quenching of the melt, are milled in the dried form and processed by any method known in the art. In the case of co-precipitation, the components are mixed in organic conditions and processed as described below.

Spray drying can be used to load the vehicle with the guest substance. The components are mixed under suitable solvent conditions and dried using precision nozzles to produce extremely uniform droplets in a drying chamber. Suitable spray drying machines include, but are not limited to, Buchi, NIRO, APV and Lab-plant spray driers used according to the manufacturer's instructions. A number of carbohydrates are unsuitable for use in spray drying as the melting points of the carbohydrates are too low, causing the dried amorphous materials to adhere to the sides of the drying chamber. Generally, carbohydrates with a melting point of less than the operating temperature of the spray drying chamber are unsuitable for use in spray drying. For example, palatinit and lactitol are not suitable for use in spray drying under conventional conditions. A determination of suitable carbohydrates can thus be made on known melting points or determined empirically. Such determinations are within the skill of one in the art.

An alternative method for manufacturing microspheres as delivery vehicles in accord with the present invention is to prepare a uniform aqueous/organic phase emulsion of the guest substance in a solution of the vehicle as the aqueous phase and a glass former in the organic phase or the converse. This is followed by drying of the emulsion droplets to form a solid solution of the guest substance and vehicle in an amorphous matrix of the glass former. In a modification of this method, the emulsion may be formed from the guest substance in solid solution in the vehicle and two different glass formers and/or polymers dissolved together in one solvent, or dissolved into two separate solvents. The solvent(s) are then removed by evaporation to yield double or multi-walled microspheres. Suitable methods for making multi-walled microspheres are described, for instance, in Pekarek et al. (1994) Nature 367:258-260; and United States Patent No. 4,861,627.

The delivery system can also be dried from an organic solution of an SP and a hydrophobic guest substance to form a glass containing homogeneously distributed guest substance in solid solution or fine suspension in the polyol glass. These glasses can then be milled and/or micronized to give microparticles of homogeneous defined sized.

The guest substance and vehicle can also be coprecipitated to give high quality powders. Coprecipitation is performed by spraying, for instance with an air brush, the various components and/or polymeric glass former into a liquid in which neither dissolves, such as ice-cold acetone.

The invention also encompasses hollow fibers for delivery of guest substances. By drawing down a heated hollow billet, fine hollow needles can be formed. These can be made to contain a finely powdered stabilized compound by introduction of the fine powder during the melting and drawing down process. The hollow fiber can also be made of thermoplastic, organic polymer and/or carbohydrate and/or HDC glass which may itself be slowly or rapidly water soluble and/or biodegradable.

An alternative embodiment of the delivery vehicle in the invention comprises a hollow vehicle comprised of poorly water soluble glass or plastic which is filled and optionally coated with SP and/or HDC glass and the guest substance. Fine hollow fibers of slowly water-soluble inorganic or organic glasses can be drawn from a hollow billet and a finely powdered SP delivery system can be incorporated into the lumen of the billet, and therefore of the fiber, during the process.

In another embodiment of the invention, coformulations of vehicles and other water soluble materials are included. For example, coformulations of vehicles with water-soluble glasses such as phosphate glasses (Pilkington Glass Company) or biodegradable plastics such as lactide or lactide/glycolide copolymers will yield a more slowly eroding vehicle for delayed release of the guest substance. To produce the coformulations, a finely powdered glass containing the guest substance can be intimately mixed with a finely powdered carboxylate glass and co-sintered. Alternatively, if a metal carboxylate glass has a lower melting point than the delivery system, the latter can be homogeneously embedded as an encapsulate in a carboxylate glass on quenching of the melt obtained. This can be milled to give a fine powder with solubilities intermediate between the relatively rapid solubility of the vehicle and the slow solubility of the carboxylate glass.

Alternative coformulations include the use of a homogeneous suspension of the finely powdered vicreous delivery system encapsulated in a carboxylate glass by drying from an organic solvent in which the carboxylate is soluble, but the amorphous powder is not, to form the carboxylate glass. This can be ground to give a fine powder which would have the relatively rapidly dissolving delivery system entrapped within a slow dissolving carboxylate glass (i.e., comparable to a conventional slow-release system). Pulsatile release formats can be achieved either by repeated encapsulation cycles using glasses of different dissolution rates, or by mixing powders of a number of coformulations with the desired range of release characteristics. Note that this glass could also be drawn or spun to give microfibers or microneedles which would be slow-release implants. It will be appreciated that any delivery system formulation should be such that it is capable of releasing the guest substance upon administration, and should not unduly effect the stability of the material being administered.

As discussed above, glasses of derivatized carbohydrates are also suitable for use herein. Suitable derivatized carbohydrates include, but are not limited to, carbohydrate esters, ethers, imides and other poorly water-soluble derivatives and polymers.

The delivery vehicle can be loaded with the guest substance by drying a solution of the guest substance containing a sufficient quantity of vehicle to form a glass on drying. This drying can be accomplished by any method known in the art, including, but not limited to, freeze drying, vacuum, spray, belt, air or fluidized-bed drying. The dried material can be milled to a fine powder before further processing the material with the polyol glass or coformulation.

Different dosing schemes can also be achieved depending on the delivery vehicle employed. A delivery vehicle of the invention can provide for a quick release or flooding dose of the guest substance after administration, where the delivery system is readily soluble. Coformulations of vehicles with slowly water soluble glasses and plastics such as phosphate, nitrate or carboxylate glasses and lactide/glycolide, glucuronide or polyhydroxybutyrate plastics and polyesters, can provide more slowly dissolving vehicles for a slower release and prolonged dosing effect. A priming and booster effect can also be realized by utilizing a hollow, slowly water soluble vehicle filled and coated with a rapidly dissolving SP and/or HDC glass loaded with the guest substance. The glass coating loaded with the guest substance will dissolve rapidly to give an initial dosing effect. There will be no dosing action while the hollow outer wall portion of the vehicle dissolves, but the initial priming dose will be followed by a booster dose of the inner filling when the hollow outer wall is breached by dissolution. Such pulsatile release format is particularly useful for delivery of immunogenic compositions. Should multiple effect pulsatile delivery be desirable, delivery vehicles with any combination of layers of "non-loaded" vehicles and vehicles loaded with the guest substances can be constructed.

The delivery of more than one guest substance can also be achieved using a delivery system comprised of multiple coatings or layers of the vehicle loaded with different materials or mixtures thereof. Administration of the solid dose delivery systems of the present invention can be used in conjunction with other conventional therapies and coadministered with other therapeutic, prophylactic or diagnostic substances.

### Methods of Delivery

The invention further encompasses methods of delivery of the solid dose systems.

Suitable delivery methods of guest substances include, but are not limited to, topical, transdermal, transmucosal, oral, gastrointestinal, subcutaneous, ocular, intramuscular, intravenous and by-inhalation (naso-pharyngeal and pulmonary, including transbronchial and transalveolar). Topical administration is, for instance, by a dressing or bandage having dispersed therein a delivery system, or by direct administration of a delivery system into incisions or open wounds. Creams or ointments having dispersed therein slow release bead or microspheres of a delivery system are suitable for use for instance as topical ointments or wound filling agents.

Compositions for transdermal administration are preferably powders of delivery systems in the form of homogeneously sized microneedles or microbeads. Larger, macroscopic needle and bead forms of the delivery systems are also provided for subdermal implantation and extended drug delivery. The particle sizes should be small enough so that they cause only minimal skin damage upon administration. The powder forms of the delivery systems can be microneedles of approximately 10-1,000 microns in length and 1-150 microns in diameter. The powders may be prepackaged in single-dose, sealed, sterile formats.

Suitable methods of transdermal administration include, but are not limited to, direct impact, ballistic, trocar and liquid jet delivery. For direct impact delivery, macroneedles can be precision-formed by methods well known in the inorganic glass forming art, such as those used for optical fibre production. These needles could be housed in a precision formed closed fitting plastic barrel and driven directly through the skin by a plunger. Ballistic administration is preferred as it is relatively painless. Generally the delivery system is accelerated in a shock wave of helium or another gas and fired into the epidermis. A suitable device for ballistic delivery is described in PCT/GB 94/00753. A suitable device for liquid-jet delivery is a Medi-ject device (Diabetes Care (1993) 1b, 1479-1484). Such liquid-jet devices are particularly useful with the larger macroneedle delivery systems which may also be delivered by the use of conventional impact ballistic devices or by trocar.

Upon transdermal administration, the degree of penetration of the delivery system can be controlled to a certain degree, not only by the ballistic microinjector, described below, but also by the shape and size of the powder particles. For example, when a relatively uniform and lesser degree of penetration is desirable, microspheres may be more suitable for the practice of the present invention. When a greater degree of penetration is desirable, a microneedle configuration may be preferred.

Because the aspect ratio (i.e., length to diameter) of the microneedles is high, they have higher masses than spherical particles with a similar diameter. If they can be induced to impact with the skin "end-on," their higher mass will give them a higher momentum for the same velocity and they will thus penetrate deeper into the tissues. When randomly oriented microneedles are put into a laminar flow of gas, they will align themselves in the direction of the air flow and in the gas-propelled ballistic injector this will ensure that they impact the skin at right angles to ensure penetration.

The delivery systems suitable for transmucosal delivery include, but are not limited to, mucoadhesive wafers, films or powders, lozenges for oral delivery, pessaries, and rings and other devices for vaginal or cervical delivery.

Compositions suitable for gastrointestinal administration include, but are not limited to, pharmaceutically acceptable powders, tablets, capsules and pills for ingestion and suppositories for rectal administration.

Compositions suitable for subcutaneous administration include, but are not limited to, various implants. Preferably the implants are macroscopic discoid, spherical or cylindrical shapes for ease of insertion and may be either fast or slow release. Since the entire implant is dissolved in the body fluids, removal of the implant is not necessary. Furthermore, the implants do not contain synthetic polymers and are biodegradable.

Compositions suitable for ocular administration include, but are not limited to microsphere and macrosphere formulations and saline drops, creams and ointments containing these and round-ended shaped rods which fit comfortably in the lower conjunctival fornix beneath the lower eyelid.

Compositions suitable for by-inhalation administration include, but are not limited to, powder forms of the delivery systems. Preferably the powders are of a particle size 0.1 to 10 microns. More preferably, the particle size is 0.5 to 5 microns. Most preferably, particle size is 1 to 4 microns. In particular for pulmonary administration, the preferred particle size is 2.5-3 microns.

Preferably SP delivery vehicle powders also contain an effective amount of a physiologically acceptable molecular water pump buffer (MWPB). A MWPB is a physiologically acceptable salt that effects a loss of water from the composition so that at ambient humidity the vapor pressure of water of crystallization is at least 14 mm Hg (2000 Pa) at 20°C and does not interfere with glass formation of the vehicle. An effective amount of an MWPB is one which sufficiently reduces hygroscopicity to prevent substantial clumping, for instance, a 50% molar ratio of potassium sulfate. Sodium sulfate and calcium lactate are the preferred salts with potassium sulfate being the most preferred.

The composite HPC delivery systems are particularly useful for by-inhalation dosage forms. For instance, 10% (w/v) αGPAC/TOAC mixed delivery systems are resistant to 95% relative humidity (RH) but recrystallize on contact with liquid water and thus release any guest substances incorporated therein. This is especially important for inhalable powders as these powders would preferably devitrify and release guest substances upon hitting liquid in the alveoli and not in the humid tracheal airways.

Atomizers and vaporizers filled with the powders are also encompassed by the invention. There are a variety of devices suitable for use in by-inhalation delivery of powders. See, e.g., Lindberg (1993) Summary of Lecture at Management Forum 6-7 December 1993 "Creating the Future for Portable Inhalers." Additional devices suitable for use herein include, but are not limited to, those described in WO9413271, WO9408552, WO9309832 and United States Patent No. 5,239,993.

Various other solid dose delivery systems are encompassed by the invention. These are suitable for delivery of a wide variety of non-medical guest substances. For instance, an HDC glass, incorporating an agricultural guest substance is dry on the shelf, even in the tropics, but releases pesticide or biological control agents on contact with liquid water on plant surfaces or in the soil. An HDC glass incorporating an enzyme is useful in adding to laundry detergents as it stabilizes the enzyme even in high humidity yet releases the enzyme immediately on contact with water. Numerous other embodiments are encompassed by the claimed invention and are within the skill of one in the art to devise.

The following examples are provided to illustrate but not limit the present invention.

### Example 1

### Methods of Making Microfiber SP Vitreous Solid Dose Delivery Systems

### a) SP microfiber formation

Glasses were formed by drying 20% solutions of either trehalose, lactitol, palatinit or GPS, containing MWPB and 1 mg/ml of the fluorescent algal protein phycoerythrin under vacuum (80 mTorr) for 16 hrs. The glasses were ground in a domestic coffee mill to yield a coarse powder which was used to fill the spinning head of a Kando K1 Kandy Floss cotton candy machine (GB Patent No. 1533012). The motor was then switched on and the powdered sugar glass heated at element settings between 5 and 9. Residence time in the spinning head was 2-10 min and a continuous process was maintained by constantly topping up the head.

The fibers produced were ground in a domestic coffee grinder and the results obtained are presented in the Table below, which shows an average of the needles produced. These data indicate that, with all three sugar glasses. reduced element settings result in the production of finer diameter microneedles. With trehalose, setting 6 gave microneedles with a mean diameter of 15 microns, and setting 9, microneedles with a mean diameter of 40 microns. With GPS, setting 9 gave microneedles with a mean diameter of 15 microns. Microneedles formed from glasses containing buffer salts remained dry at ambient temperatures and humidities. Microneedles containing phycoerythrin showed retention of biological activity as assessed by fluorescence.

| Microneedle size analysis | | |
|---|---|---|
| | | |
| | Length(µm) | Width(µm) |
| | | |
| Mean | 192.60 | 43.35 |
| Standard Error | 12.53 | 2.33 |
| Median | 167.5 | 37.5 |
| Mode | 137.5 | 47.5 |
| Standard Deviation | 123.44 | 22.91 |
| Sample Variance | 15237.75 | 524.72 |
| Kurtosis | 16.17 | 2.55 |
| Skewness | 3.35 | 1.45 |
| Range | 862.5 | 115 |
| Minimum | 67.5 | 10 |
| Maximum | 930 | 125 |
| Sum | 18682.5 | 4205 |
| Count | 97 | 97 |
| Confidence Level (95.000%) | 24.57 | 4.56 |

### b) Binary SP/organic composite glass microfiber formation

Glasses were formed by drying a 5:1:1 mixture of trehalose, sodium octanoate and water under vacuum (80 mTorr) for 16 hrs. The glasses were ground in a domestic coffee mill to yield a coarse powder which was used to fill the spinning head of a Kando K1 Kandy Floss machine. The motor was then switched on and the powdered binary carbohydrate/organic glass heated at element settings between 5 and 9. As with pure trehalose glasses, reduced element settings resulted in the production of finer diameter microneedles. The binary mixture glasses can be tailored to yield glasses with significantly different tensile properties compared to the corresponding pure trehalose glasses. Residence time in the spinning head was again 2-10 min and a continuous process was maintained by constantly topping up the head. The results obtained indicate that variations of the melting points and dissolution times of the glasses and the resulting physical properties of the microfibers can be achieved by varying both the carbohydrate/organic molecules and ratios used.

### Example 2

### Methods of making Powder SP Vitreous Solid Dose Delivery Systems

### a) Incorporation of active in SP vitreous deliver vehicle to yield micronized powders

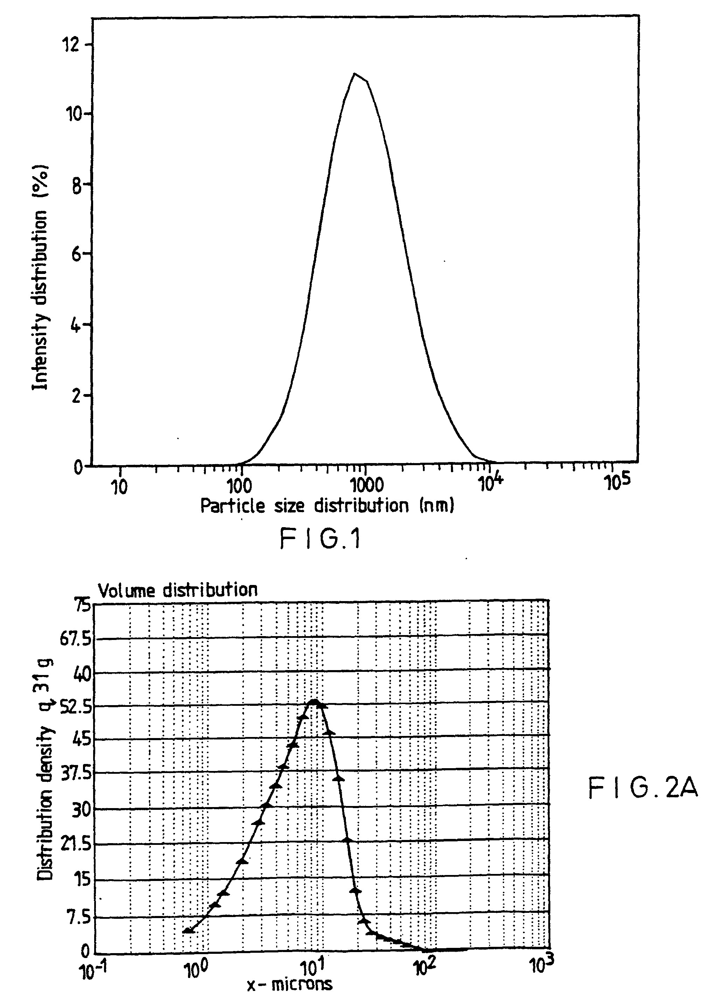

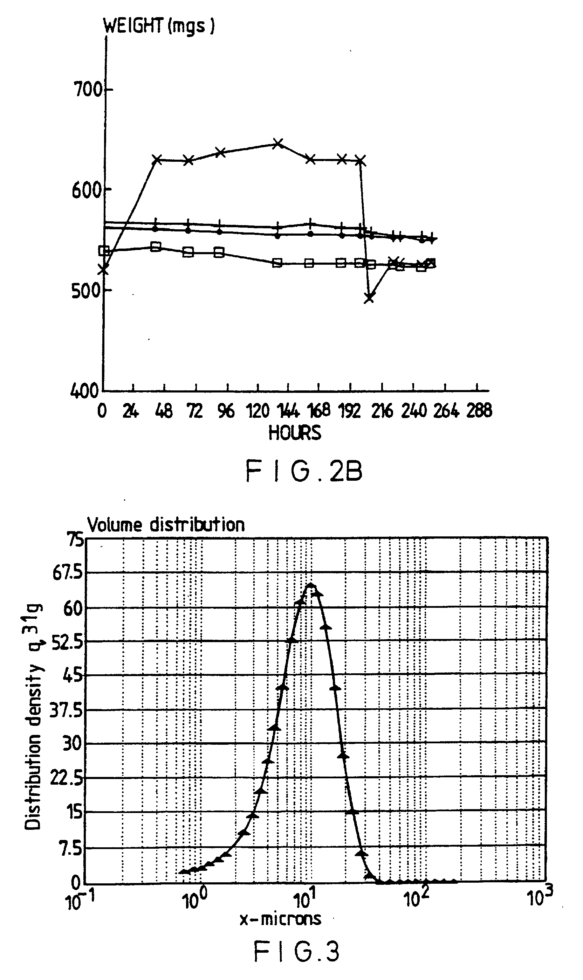

Glasses were formed by drying 20% solutions of either trehalose, lactitol, palatinit, GPM or GPS, containing an equimolar ratio of MWPB and protein, by freeze-drying under vacuum (80 mTorr) for 16 hrs. The glasses were powdered using a Trost air-jet mill. Particle size in the micronized powders were measured using a Malvern Mastersizer laser particle sizer. The results obtained with micronized powders obtained from an original solution of 0.5 M trehalose and 0.5 M calcium lactate showed a monodisperse particle distribution with mean particle diameters of 1.1 microns (Figure 1). The powders containing MWPB remained a free-flowing powder and showed no change in particle size or clumping and uptake of water on extended exposure to ambient temperatures and humidities (Figures 2A and 2B).

### b) Incorporation of active in SP vitreous delivery vehicle to yield spray-dried powders

20% solutions of trehalose containing MWPB salts and protein (phycoerythrin) were dried in a Buchi or Lab-Plant spray drier at a pump speed of 500-550 ml/hr and an inlet temperature of 180°C. Particle size was measured using a SympaTec laser particle sizer. The spray-dried powders showed a monodisperse particle distribution with a sufficiently narrow peak size distribution for effective use as particles in a powder ballistic device. In the results shown in Figure 3, particle size analysis of a spray-dried powder produced by spray drying a mixture of 0.5 M trehalose and 0.5 M calcium lactate on a Lab-Plant spray drier showed a mean particle diameter of 8.55 microns and illustrates the tight peak distribution obtained.

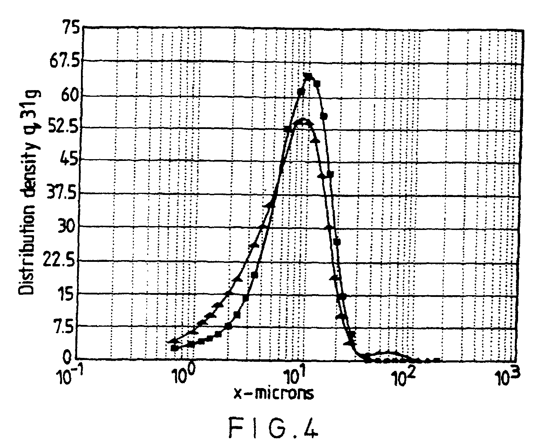

Variation of the mean particle size can be achieved by varying either the composition of the mixture to be spray dried or the characteristics of the spray drier nozzle assembly used. The results shown in Figure 4 provide a comparison of the particle size analysis of the spray-dried powder as in Figure 3 with a spray-dried powder produced by drying the same mixture on the Buchi spray drier which uses a different nozzle assembly. The peak distribution shown in Figure 4 shows an equally narrow range but the mean particle size is now 7.55 microns.

These data show that the particles obtained by different spray-drying processes are equally suitable to provide compositions for ballistic delivery. Note that the ability to vary particle size results in compositions with different penetrative characteristics. This is particularly important for determining intradermal, intramuscular, intravenous or intramuscular delivery as the penetration is a function of particle momentum and the distribution is a function of the scatter of particle size.

### c) Incorporation of active in SP vitreous delivery vehicle by drying from organic solvents

A 50 mg/ml solution of CSA in a 1.1 mixture of ethanol:water, containing 20% trehalose, was air-dried at ambient temperature to form a clear trehalose glass containing CSA in solid suspension or solution. The glass was ground to give a powder, according to the method described in Example 1, and remained a free-flowing powder at ambient temperature and humidities. Addition of the powder to water resulted in the dissolution of the trehalose and the formation of a uniform aqueous suspension of CSA.

### d) Incorporation of active in SP vitreous delivery vehicle by co-precipitation

20% solutions of trehalose, lactitol, palatinit, GPM or GPS, containing MWPB and protein (phycoerythrin) were dried by spraying into an acetone-solid carbon dioxide freezing bath. The precipitated powders were separated by centrifugation or filtration and air dried to remove residual solvent. The powders again showed a monodisperse particle distribution and those containing buffer formulation salts remained dry at ambient temperatures and humidities.

### e) Formation of composite vitreous solid dose delivery vehicle of hydrophobic active in SP by drying from organic solvents

Two different solvent systems were used to produce composite glasses. In the first case, CSA was dissolved in absolute ethanol and an equal volume of water was then added slowly so that the CSA which precipitated on each addition was allowed to redissolve. Trehalose was then dissolved in the 50% v/v ethanol solution to a final concentration of 50% w/v. Composite glasses were produced by evaporating the mixed solvent on a hotplate at 70°C. In the second case, CSA and trehalose were both dissolved in DMF and again the composite glass was made by evaporation as described above. In both cases, a slightly opalescent glass resulted. Drops of water were then overlaid on the glass films to study the dissolution and release properties of the glasses.

The results obtained indicate that the glasses behaved remarkably differently. Glasses made from DMF were water repellent with an obviously hydrophobic surface. They gradually developed opaque white patches and clumps of precipitated CSA where they were in contact with water. Glasses made from 50% ethanol were hydrophilic. They dissolved rapidly in the water and in doing so they released a cloud of very fine CSA particles. This latter glass appeared to contain CSA in either a fine solid suspension or a solid solution in the trehalose glass which released the CSA as a precipitate when the trehalose dissolved. As such, it represents a very useful dosage form for CSA with high bioavailability due to its uniform and finely divided format after release.

The different behavior of glasses of identical composition after drying from different solvents suggests an interesting and useful process providing precise control over the pattern of deposition of the different glasses during solvent evaporation. Since CSA is more soluble in DMF than is trehalose, composite glasses of 10-20% CSA in trehalose prepared from this solvent tend to have hydrophilic trehalose cores and hydrophobic CSA coatings. In contrast, when 50% ethanol evaporates, the early loss of ethanol in the 97% azeotrope causes CSA to come out of solution surrounded by trehalose syrup which then solidifies as the continuous phase leading to a CSA in trehalose glass solid emulsion.

### Example 3

### Coformulation of vitreous solid dose delivery system of composite SP and organic glasses by evaporation

Microparticles of trehalose containing MB9 were prepared by spray drying as described in Example 2b. The solution dried contained 0.39 M trehalose and 0.14 M. calcium lactate and 0.5% MB9. These particles were coated by adding them to a saturated solution of zinc palmitate (ZnC₁₆) in toluene and cooling from 60°C to 30°C. This deposited a layer of ZnC₁₆ on the particles which were then filtered under vacuum to remove the excess ZnC₁₆, washed with acetone and air-dried. The resulting powder remained unwetted in water for at least three days (the particles floated in the water without sinking or releasing MB9 and thereafter slowly released dye into the water). Thus, otherwise water soluble powders may be made water impermeable by coating with metal carboxylates such as ZnC₁₆ to yield slow release formats. Note that the coating material is most likely in crystalline form and not a glass; therefore, the solid phase in which the guest substances are suspended need not be in the glass phase to be water impermeable.

## Claims

1. A particulate composition, for therapeutic use, suitable for administration by inhalation, wherein the particles consist of a solid solution comprising a therapeutic agent and a glass-forming carbohydrate, higher than a monosaccharide, and that is capable of stabilising the agent during spray-drying and storage, with the proviso that when the therapeutic agent is insulin, the solution does not comprise citrate.

2. A composition according to claim 1, wherein the carbohydrate is selected from disaccharides, trisaccharides and oligosaccharides, the corresponding sugar alcohols, polysaccharides and chemically modified carbohydrates.

3. A composition according to claim 1, wherein the carbohydrate is a non-reducing glycoside of a polyhydroxy compound selected from sugar alcohols and other straight chain polyalcohols.

4. A composition according to claim 1, wherein the carbohydrate is trehalose, maltose, lactose, maltulose, isomaltulose, lactulose, raffinose, stachyose, melezitose, dextran, maltitol, lactitol, α-D-glucopyranosyl-1→6-sorbitol, α-D-glucopyranosyl-1→6-mannitol or palatinit.

5. A composition according to claim 1, wherein the carbohydrate is trehalose.

6. A composition according to any preceding claim, wherein the particles are 0.1 to 10 µm in size.

7. A composition according to claim 6, wherein the particles are 1 to 4 µm in size.

8. A composition according to any preceding claim, wherein the therapeutic agent is a protein or peptide, nucleotide, oligonucleotide or nucleic acid.

9. A composition according to any of claims 1 to 7, wherein the therapeutic agent is an enzyme, a growth hormone, a growth factor, a monoclonal antibody, an interferon, an interleukin or a cytokine.

10. A composition according to any of claims 1 to 7, wherein the therapeutic agentis cyclosporin A, estrogen, progesterone, testosterone, estradiol, SH-135 or tamoxifen.

11. A composition according to claim 8, wherein the therapeutic agent is insulin.

12. A composition according to any preceding claim, without the proviso, wherein the particles further comprise a physiologically acceptable carboxylate, nitrate, sulfate or bisulfate glass.

13. A composition according to any preceding claim, without the proviso, wherein the particles further comprise an inhibitor of the Maillard reaction.

14. A composition according to any preceding claim, without the proviso, wherein the particles further comprise a molecular water pump buffer.

15. A composition according to any of claims 1 to 11, without the proviso, wherein the particles consist solely of the therapeutic agent, the carbohydrate and, optionally, a Maillard reaction inhibitor or a molecular water pump buffer.

16. A device for the pulmonary delivery of a therapeutic agent, wherein the device includes particles as defined in any of claims 1 to 15.

17. A method of making particles as defined in any of claims 1 to 15, without the proviso, comprising the steps of melting the glass-forming carbohydrate, incorporating the therapeutic agent therein, wherein the melt temperature is sufficient to fluidise the carbohydrate but insufficient to substantially inactivate the therapeutic agent, quenching the melt, and milling the quenched product.

18. A method of making particles as defined in any of claims 1 to 15, comprising the steps of dissolving or suspending the carbohydrate and the therapeutic agent in a solvent, and drying the resultant solution or suspension to form particles.

19. A method according to claim 18, wherein the drying comprises spray-drying.

20. A method according to claim 18, without the proviso, wherein the drying comprises supercritical fluid evaporation.

## Patentansprüche

1. Teilchenförmige Zusammensetzung für eine therapeutische Verwendung, die für eine Verabreichung durch Inhalation geeignet ist, wobei die Teilchen aus einer festen Lösung, umfassend ein therapeutisches Mittel und ein glasbildendes Kohlenhydrat, das höher als ein Monosaccharid ist und das in der Lage ist, das Mittel während einer Sprühtrocknung und Lagerung zu stabilisieren, bestehen, mit der Maßgabe, dass, wenn das therapeutische Mittel Insulin ist, die Lösung kein Citrat umfasst.

2. Zusammensetzung nach Anspruch 1, wobei das Kohlenhydrat aus Disacchariden, Trisacchariden und Oligosacchariden, den entsprechenden Zuckeralkoholen, Polysacchariden und chemisch modifizierten Kohlenhydraten ausgewählt wird.

3. Zusammensetzung nach Anspruch 1, wobei das Kohlenhydrat ein nicht-reduzierendes Glycosid einer Polyhydroxyverbindung, ausgewählt aus Zuckeralkoholen und anderen geradkettigen Polyalkoholen, ist.

4. Zusammensetzung nach Anspruch 1, wobei das Kohlenhydrat Trehalose, Maltose, Lactose, Maltulose, Isomaltulose, Lactulose, Raffinose, Stachyose, Melezitose, Dextran, Maltitol, Lactitol, α-D-Glucopyranosyl-1→6-sorbitol, α-D-Glucopyranosyl-1→6-mannitol oder Palatinit ist.

5. Zusammensetzung nach Anspruch 1, wobei das Kohlenhydrat Trehalose ist.

6. Zusammensetzung nach Anspruch 1, wobei die Teilchen 0,1 bis 10 µm groß sind.

7. Zusammensetzung nach Anspruch 6, wobei die Teilchen 1 bis 4 um groß sind.

8. Zusammensetzung nach einem beliebigen vorangegangenen Anspruch, wobei das therapeutische Mittel ein Protein oder Peptid, Nukleotid, Oligonukleotid oder eine Nukleinsäure ist.

9. Zusammensetzung nach einem der Ansprüche 1 bis 7, wobei das therapeutische Mittel ein Enzym, ein Wachstumshormon, ein Wachstumsfaktor, ein monoklonaler Antikörper, ein Interferon, ein Interleukin oder ein Zytokin ist.

10. Zusammensetzung nach einem der Ansprüche 1 bis 7, wobei das therapeutische Mittel Cyclosporin A, Östrogen, Progesteron, Testosteron, Östradiol, SH-135 oder Tamoxifen ist.

11. Zusammensetzung nach Anspruch 8, wobei das therapeutische Mittel Insulin ist.

12. Zusammensetzung nach einem beliebigen vorangegangenen Anspruch ohne die Maßgabe, wobei die Teilchen des weiteren ein physiologisch annehmbares Carboxylat-, Nitrat-, Sulfat- oder Bisulfat-Glas umfassen.

13. Zusammensetzung nach einem beliebigen vorangegangenen Anspruch ohne die Maßgabe, wobei die Teilchen des weiteren einen Inhibitor der Maillard-Reaktion umfassen.

14. Zusammensetzung nach einem beliebigen vorangegangenen Anspruch ohne die Maßgabe, wobei die Teilchen des weiteren eine molekulare Wasser-Pump-Puffersubstanz umfassen.

15. Zusammensetzung nach einem der Ansprüche 1 bis 11 ohne die Maßgabe, wobei die Teilchen allein aus dem therapeutischen Mittel, dem Kohlenhydrat und gegebenenfalls einem Inhibitor der Maillard-Reaktion oder einer molekularen Wasser-Pump-Puffersubstanz bestehen.

16. Vorrichtung zur pulmonalen Abgabe eines therapeutischen Mittels, wobei die Vorrichtung Teilchen wie in einem der Ansprüche 1 bis 15 definiert umfasst.

17. Verfahren zur Herstellung von Teilchen wie in einem der Ansprüche 1 bis 15 definiert ohne die Maßgabe, welches die Schritte umfasst, das glasbildende Kohlenhydrat zu schmelzen, das therapeutische Mittel dort hinein einzuarbeiten, wobei die Schmelztemperatur ausreichend ist, um das Kohlenhydrat zu fluidisieren, aber nicht ausreichend ist, um das therapeutische Mittel substantiell zu inaktivieren, die Schmelze schnell abzukühlen und das schnell abgekühlte Produkt zu mahlen.

18. Verfahren zur Herstellung von Teilchen wie in einem der Ansprüche 1 bis 15 definiert, welches die Schritte umfasst, das Kohlenhydrat und das therapeutische Mittel in einem Lösemittel zu lösen oder zu suspendieren und die resultierende Lösung oder Suspension zu trocknen, um Teilchen zu bilden.

19. Verfahren nach Anspruch 18, wobei das Trocknen eine Sprühtrocknung umfasst.

20. Verfahren nach Anspruch 18 ohne die Maßgabe, wobei das Trocknen das Verdampfen eines überkritischen Fluids umfasst.

## Revendications

1. Composition particulaire, pour une utilisation thérapeutique, appropriée pour une administration par inhalation, dans laquelle les particules consistent en une solution solide comprenant un agent thérapeutique et un hydrate de carbone vitrifiant, supérieur à un monosaccharide, et qui est capable de stabiliser l'agent durant le séchage par pulvérisation et la conservation, avec la condition que, lorsque l'agent thérapeutique est l'insuline, la solution ne comprend pas de citrate.

2. Composition selon la revendication 1, dans laquelle l'hydrate de carbone est choisi parmi les disaccharides, trisaccharides et oligosaccharides, les alcools glucidiques, polysaccharides et hydrates de carbone modifiés chimiquement correspondants.

3. Composition selon la revendication 1, dans laquelle l'hydrate de carbone est un glucoside non-réducteur d'un composé polyhydroxylé choisi parmi les alcools glucidiques et autres polyalcools à chaîne droite.

4. Composition selon la revendication 1, dans laquelle l'hydrate de carbone est un tréhalose, un maltose, un lactose, un maltulose, un isomaltulose, un lactulose, un raffinose, un stachyose, un mélézitose, un dextran, un maltitol, un lactitol, un α-D-glucopyranosyl-1→6-sorbitol, un α-D-glucopyranosyl-1→6-mannitol ou un palatinite.

5. Composition selon la revendication 1, dans laquelle l'hydrate de carbone est un tréhalose.

6. Composition selon l'une quelconque des revendications précédentes, dans laquelle les particules ont une taille de 0,1 à 10 µm.

7. Composition selon la revendication 6, dans laquelle les particules ont une taille de 1 à 4 µm.

8. Composition selon l'une quelconque des revendications précédentes, dans laquelle l'agent thérapeutique est une protéine ou un peptide, un nucléotide, un oligonucléotide ou un acide nucléique.

9. Composition selon l'une quelconque des revendications 1 à 7, dans laquelle l'agent thérapeutique est une enzyme, une hormone de croissance, un facteur de croissance, un anticorps monoclonal, un interféron, une interleukine ou une cytokine.

10. Composition selon l'une quelconque des revendications 1 à 7, dans laquelle l'agent thérapeutique est la cyclosporine A, un estrogène, la progestérone, la testostérone, l'estradiol, le SH-135 ou le tamoxifène.

11. Composition selon la revendication 8, dans laquelle l'agent thérapeutique est l'insuline.

12. Composition selon l'une quelconque des revendications précédentes, sans la condition, dans laquelle les particules comprennent en plus un verre de carboxylate, de nitrate, de sulfate ou de bisulfate physiologiquement acceptable.

13. Composition selon l'une quelconque des revendications précédentes, sans la condition, dans laquelle les particules comprennent en plus un inhibiteur de la réaction de Maillard.

14. Composition selon l'une quelconque des revendications précédentes, sans la condition, dans laquelle les particules comprennent en plus un tampon de pompe à eau moléculaire.

15. Composition selon l'une quelconque des revendications 1 à 11, sans la condition, dans laquelle les particules consistent seulement en l'agent thérapeutique, l'hydrate de carbone et, optionnellement, un inhibiteur de la réaction de Maillard ou un tampon de pompe à eau moléculaire.

16. Dispositif pour la libération pulmonaire d'un agent thérapeutique, dans lequel le dispositif inclut des particules comme cela est défini dans l'une quelconque des revendications 1 à 15.

17. Procédé de production de particules comme cela est défini selon l'une quelconque des revendications 1 à 15, sans la condition, comprenant les étapes de fonte de l'hydrate de carbone vitrifiant, d'incorporation de l'agent thérapeutique à celui-ci, dans lequel la température de fonte est suffisante pour fluidifier l'hydrate de carbone, mais insuffisante pour substantiellement inactiver l'agent thérapeutique, de refroidissement brusque de la phase vitreuse, et de broyage du produit refroidi.

18. Procédé de production de particules comme cela est défini dans l'une quelconque des revendications 1 à 15, comprenant les étapes de dissolution ou de mise en suspension de l'hydrate de carbone et de l'agent thérapeutique dans un solvant, et de séchage de la solution ou de la suspension résultante pour former des particules.

19. Procédé selon la revendication 18, dans laquelle le séchage comprend un séchage par pulvérisation.

20. Procédé selon la revendication 18, sans la condition, dans laquelle le séchage comprend l'évaporation d'un fluide supercritique.
